# EUROPEAN PATENT SPECIFICATION

(11) **EP 0 731 718 B1**
(45) Date of publication and mention of the grant of the patent: **14.08.2002**
(21) Application number: 94903401.1
(22) Date of filing: 01.12.1993
(51) Int. Cl.: A61B 17/34

(54) **TROCAR WITH UNIVERSAL SEAL PROTECTION**
TROCAR MIT EINEM UNIVERSELLEN DICHTUNGSSCHUTZ
TROCART AVEC UNE PROTECTION DE JOINT UNIVERSEL

(43) Date of publication of application: 18.09.1996
(73) Proprietor: Applied Medical Resources Corporation, Rancho Santa Margarita, CA 92688 (US)
(72) Inventor: GADBERRY, Donald, L., San Juan Capistrano, CA 92675 (US)
(74) Representative: Enskat, Michael Antony Frank
(86) International application number: US9311694
(87) International publication number: WO9515189

(56) References cited:
- EP-A- 0 567 142
- WO-A-94/07552
- US-A- 1 800 045
- US-A- 4 387 879
- US-A- 5 176 652
- US-A- 5 209 737

## Description

This invention relates to trocars and to methods of protecting valves within trocars against instruments operable through a working channel in the trocar.

Mechanical trocars typically includes a cannula defining a working channel, and a housing which encloses valves that function to inhibit the escape of insufflation gases. The cannula of the trocar is adapted to be positioned across the abdominal wall of a patient using a obturator which is initially inserted into the working channel and then removed once the cannula is in place. Various elongate instruments can be inserted through the working channel of the trocar to reach, and perform operative functions, at a site within the abdomen. It is the function of the valves to engage the outer surface of such an instrument and form seals around the instrument to prevent the escape of insufflation gases.

Trocar valves are commonly formed from elastomeric materials which are highly susceptible to puncture and tearing by sharp instrument configurations. Since many instruments, and of course the obturator, typically have sharp distal tips, it has become particularly desirable to protect the valves from these objects.

In some cases the valves have been operable by lever arms which have provided the valves with variable diameters. In such a configuration, protective shields have been provided for attachment to the obturator. These shields have functioned with the lever arms to expand the valve. Shields have also been designed to extend between the seal and the sharp distal tip of the obturator. Such shields have been adapted solely for use with obturators and are not necessarily applicable to other types of instruments.

Other valve protectors have functioned relative to the lever arms in response to insertion of any instrument into the working channel. Such a mechanism is disclosed in applicant's US patent no 5,308,336 (application serial no 07/952,300 filed on September 28, 1992 and entitled Seal Protection Mechanism) and in US Patent No 5,209,737.

Also, US Patent No 4,387,879 discloses a valve assembly for introducing instruments into body cavities and comprising a conical seal and conical retainers. The conical retainers prevent unwarranted contact of the instrument with the seal. European Patent Specification No 0 567 142 discloses a longitudinal guiding member for opening a seal.

In spite of these advances in the art, it has remained desirable to provide a mechanism which can protect an ordinary septum valve devoid of any lever mechanism and also to limit friction on the instrument during axial passage through the valve. In the absence of the protection mechanism for the ordinary septum seal, instruments having sharp tips have been free to engage the valve often resulting in damage to the valve which renders the entire trocar inoperable. Even in those instances where the valve is not cut or otherwise damages, large instruments which are required to move through a small orifice in a septum valve have experienced significant friction forces making axial movement of the instrument difficult.

In accordance with the present invention, a trocar is provided with a valve expander which is movable from a first position spaced from the valve to a second position in proximity to the valve. The valve expander has a first configuration in a relaxed state and a second configuration in an expanded state. When an instrument is inserted into the trocar, it engages the valve expander at the first position and moves it axially into proximity with the valve. When the expander initially contacts the valve, it is generally in its relaxed state. Further axial movement of the instrument causes the expander to move the remainder of its stroke to the second position while at the same time moving the valve radially outwardly enlarging the orifice of the valve. In this second position, the valve expander is disposed between the instrument and at least a portion of the valve.

Once the instrument is operatively disposed it is necessary to move the valve adjuster in order to permit the valve to form a seal with the instrument. This can be accomplished by withdrawing the instrument slightly to move the adjuster from the second position back to the first position. This movement may be assisted by a spring which biases the adjuster to the first position.

In a first aspect of the invention, there is provided a trocar adapted to provide a working channel across a body wall to accommodate a surgical instrument having a particular diameter, the trocar comprising: a cannula defining the working channel along an axis extending between a distal end and a proximal end of the cannula; a housing attached to the cannula at the proximal end of the cannula; a valve disposed in the housing so that when engaged by the instrument it forms a fluid tight seal across the working channel; a valve adjustment means disposed within the housing and having at least a portion disposed in the working channel proximally of the valve, the valve adjustment means being adapted to move axially within said working channel between a first position spaced from the valve and a second position wherein at least a portion of the valve adjustment means is interposed between the valve and a portion of the working channel which extends through the valve, such that the valve is protected from the instrument when the distal tip of the instrument is inserted there through; and the valve adjustment means being responsive to insertion of the instrument into the working channel to move from the first position to the second position.

In a second aspect of the invention there is provided a method for protecting a valve disposed in a trocar having an axis, the trocar having a working channel extending through an orifice in the valve the method comprising the steps of: providing in the trocar, a valve adjuster movable from a first position spaced from the valve to a second position in contact with the valve, the valve adjuster in the second position being disposed to enlarge the orifice of the valve; inserting an instrument into the working channel of the trocar, the instrument having an elongate shaft and a sharp or puncture prone configuration; during the inserting step engaging the valve adjuster at the first position with the instrument; moving the valve adjuster axially with the instrument from the first position to the second position; and radially expanding the valve adjuster and the valve to enlarge the orifice of the valve.

Trocars and methods of protecting a valve within a trocar each embodying the invention, will now be described with reference to the accompanying diagrammatic drawings, in which:
Fig. 1 is a side elevation view of a trocar with its cannula disposed in an operative position across the body wall;
Fig. 2 is an axial cross-section view of a valve housing associated with the trocar of Fig. 1, a valve adjuster in the housing is illustrated in a first position and a relaxed state;
Fig. 3 is an axial cross-section view similar to Fig. 2 wherein the valve adjuster is illustrated in a second position and an expanded state; and
Fig. 4 is an axial cross-section view of a further embodiment of the invention including a single valve and an associated valve adjuster.

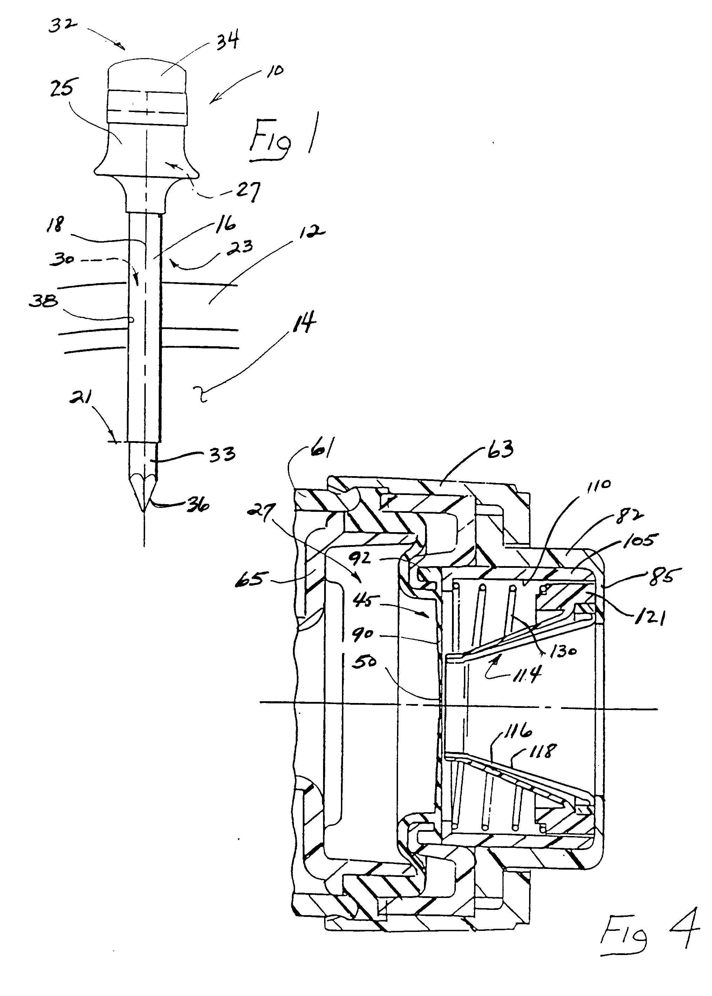

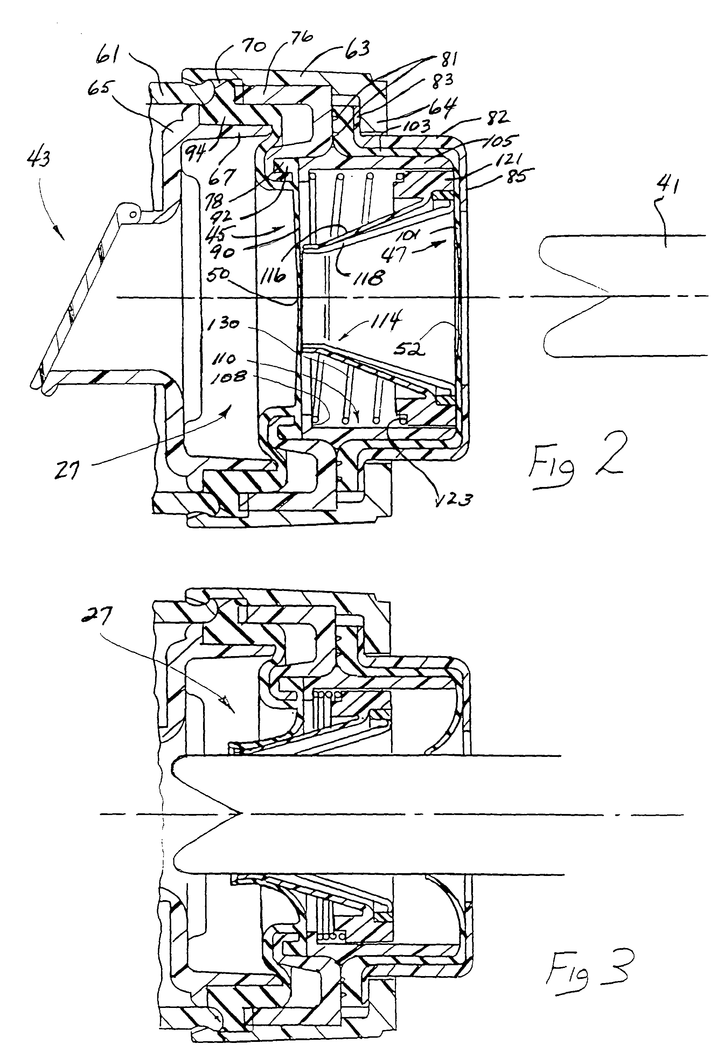

A trocar is illustrated in Figure 1 and designated generally by the reference numeral 10. The purpose of the trocar 10 is to establish a working channel across a body wall, such as the abdominal wall 12. The trocar 10 extends from regions exterior of the body to regions interior of the body, such as the abdominal cavity 14. The trocar 10 is commonly used in laparoscopic surgery wherein the abdominal cavity 14 is pressurized with an insufflation gas in order to provide for organ separation and otherwise increase the size of the operative environment.

The trocar 10 includes a cannula 16 which extends along an axis 18 between a distal end 21 and a proximal end 23. At the proximal end 23, a housing 25 is attached to the cannula 16 and provides an enclosure for a valve mechanism 27 which is described in greater detail below. When the trocar 10 is operatively disposed, these elements (the cannula 16, the housing 25, and valve mechanism 27) define a working channel 30 into the cavity 14. Various instruments, such as scopes, clamps and staplers, can be inserted through this channel 30 to perform operative functions in the cavity 14. These instruments, which will typically have elongate shafts and sharp distal tips, are collectively represented by the instrument 41 in Figure 2.

Placing the cannula 16 in its operative position is accomplished by using an obturator 32 which has an elongate spike or shaft 33 with a cap 34 disposed at its proximal end. Of importance to the present invention is a sharp tip 36 which is disposed at the distal end of the obturator shaft 33. It is the purpose of the obturator 32 to place the cannula 16 in its operative position across the abdominal wall 12. To accomplish this function, the obturator 32 is initially loaded into the working channel 30 by moving the sharp tip 36 through the seal mechanism 27 in the housing 25. With the sharp tip 36 disposed distally of the cannula 16, the obturator 32 and the cannula 16 are forced through the abdominal wall 12 to create a puncture or hole 38. Once the hole 38 has been created and the cannula 16 is in its operative position, as illustrated in Figure 1, the obturator 32 can be removed from the working channel 30 through the housing 25 and the seal mechanism 27.

Once the trocar 10 is operatively disposed and the obturator 32 has been removed, additional insufflation gases can be introduced into the cavity 14 to enlarge the surgical environment. The insufflation gas, which commonly includes carbon dioxide, can be introduced through the working channel 30 of the trocar 10. Laparoscopic instruments which include retractors scalpels, staplers, and clamps, for example, are all represented by the instrument 41 illustrated in Figure 2 and 3. Such instruments will typically have an elongate cylindrical configuration and may have a sharp distal tip.

It is the purpose of the seal mechanism 27 to block the escape of insufflation gasses from the cavity 14 through the working channel 30 of the trocar 10. This blockage is required not only when the instrument 41 is absent from the working channel 30, but when the instrument 41 is operatively disposed within the trocar 10.

Referring now to Figure 2, the seal mechanism 27 can take many different forms, but in the illustrated embodiment, a flapper valve 43 is provided along with a pair of septum valves 45 and 47. Each of the septum valves 45 and 47 extends generally radially of the axis 18 and defines a hole or orifice 50 and 52 respectively, along the working channel 30. It is these valves 45, 47 which are intended to engage the outer surface of the obturators 32 or instrument 41, in order to form a seal across the working channel. Since these instruments have various diameters, it is important that the valves 45, 47 be formed of elastomeric material so that the respective orifices 50, 52 can vary in size.

The greater the range of instrument diameters to be accommodated, the greater the need for compliance in the valves 45, 47. This compliance generally results from the elastomeric characteristics of the valves 45, 47. Where a particularly large range of instrument diameters is to be accommodated, it is customary to provide multiple valves each having a different orifice size. Such is the case with the embodiment of Figure 2 where the orifice 52 of the valve 47 is about 6mm in diameter and the orifice 50 of the valve 45 is about 3mm in diameter.

The greater the requirement for diameter accommodation or compliance, the greater the need for elasticity in the material forming the valves 45, 47. But this elasticity also makes the valves 45, 47 particularly susceptible to sharp edges and points which are typically associated with the obturator 32 and other instruments, such as the instrument 41.

In a preferred embodiment, the seals 45, 47 are mounted within the housing 25 by several elements each of which has an annular configuration and a cross-sectional shape as illustrated in Figures 2 and 3. For example, the housing 25 may include a body member 61, and a cap 63 which is fixed (for example with a snap fit) to the body member 61. In the illustrated embodiment, the cap 63 has a radial flange 64. Generally interiorly of the member 61 and the cap 63 are several elements which function to mount the flapper valve 43 and the septum valves 45, 47 to perform their respective functions. In the illustrated embodiment, the flapper valve 43 is mounted on a flapper valve support 65 which includes an annular flange 67. This flange 67 defines a cavity 70 with the body member 61 and cap 63.

A septum valve support 76 is disposed proximally of the valve support 65 and includes portions defining an axial groove 78. The septum valve support 76 also includes a pair of annular projections 81 which extend axially proximally within the housing 25. Seated within the flange 64 of the cap 63 is a cover 82 having an outwardly extending flange 83 and an inwardly extending flange 85.

The body member 61 and cap 63 together with the supports 65, 76 and the cover 82 are all formed from a rigid material such as an injection molded plastic. It is the purpose of these elements to house the valves 43, 45 and 47 and to support these valves in their function to maintain insufflation pressures. This function includes not only the capability of forming a seal with the obturator 32 or instrument 41, but also to form a seal with the housing 25.

A configuration of particular interest for the valve 45 is illustrated in Figure 2. This embodiment of the valve 45 includes not only a septum 90, but also an annular flange 92 which is seated within the axial groove 78 of the seal support 76. Integral with the septum 90 is an extension 94 of the valve 45 which extends radially outwardly and terminates in the annular cavity 70. When the cap 63 is forced into the body member 61, this extension 94 is compressed between the member 61, the cap 63 and the valve support 65. In this manner, the valve 45 forms a seal with the housing 25 of the trocar 10.

The valve 47 also includes a septum 101 and an integral extension 103 which is disposed inwardly of the cover 82 and is sandwiched between the cover 82 and the valve support 76 when the cap 63 is snap fit to the body member 61. In this location, the extension 103 is forced against the annular projections 81 thereby forming a seal between the housing 25 and the valve 47.

Of particular interest to the present invention is a separator 105 which is disposed between the septums 90 and 101 of the respective valves 45 and 47. The separator 105 has a generally cylindrical configuration, one end of which abuts the septum 90 maintaining the flange 92 within the axial groove 78. The other end of the separator 105 abuts the septum 101 maintaining the valve 47 against the inner surface of the cover 82. This separator 105 includes a proximally facing shoulder 108 which defines in part a guideway 110 that extends axially of the trocar 10 and has an annular configuration in the illustrated embodiment.

As previously noted, the orifice 50 associated with the valve 45 is smaller than the orifice 52 associated with the valve 47. Thus the septum 90 of the valve 45 extends inwardly further than the septum 101 of the valve 47. As a consequence, an instrument having a relatively small diameter may pass through the orifice 52 without touching the septum 101. However, it is intended that such an instrument with a smaller diameter would not pass through the orifice 50 and would therefore form a seal with a septum 90. In this instance, the valve mechanism 27 relies upon the valve 45 to form a seal with the instrument 41.

A problem addressed by the present invention occurs when an instrument, such as the instrument 41, with the diameter larger than the orifice 52 is inserted into the trocar 10. In this instance, the instrument 41 would contact the valve 47 and form the desired seal with the septum 101. As the instrument 41 is inserted further into the trocar 10, it might also contact the septum 90 due to the smaller orifice 50. Under these circumstances, a significant portion of the septum 90 would engage the outer surface of the instrument 41 creating undesirable friction or drag which would hinder the axial movement of the instrument 41. Of equal importance is the desirability to protect the valve 45 from any sharp configuration which may be associated with the obturator 32 or instrument 41. These functions are accomplished in a preferred embodiment by enlarging the orifice 50 formed by the septum 90 of the valve 45.

In a preferred embodiment a valve adjuster 114 is provided with an interleaved configuration such as that disclosed in applicant's US Patent No 5,308,336. This patent discloses a structure including a pair of outer leaves 116 which alternate with a pair of inner leaves 118 to form an expandable funnel. The outer leaves 116 are mounted on an annular support 121 which includes a distally facing shoulder 123. This support 121 is slidable within the guideway 110 between a first position in proximity to the valve 47, as illustrated in Figure 2, and a second position in proximity to the valve 45 as illustrated in Figure 3.

In the first position, the valve adjuster 114 will be in a relaxed state with the leaves 116 and 118 extending inwardly from the valve 47 toward the valve 45, to a radius typically not less than the radius of the orifice 52. With this configuration, an instrument smaller than the orifice 52, such as a 5mm instrument, would contact neither the septum 101 nor the leaves 116, 118, but would contact the septum 90 and form a seal with the valve 45.

When an instrument having a diameter larger than the orifice 52 is inserted into the working channel 30, it initially contacts the septum 101 to form a seal with the valve 47. Further axial movement of the instrument 41 engages the leaves 116, 118 in the relaxed state and begins to move the valve adjuster 114 axially distally along the guideway 110. With further axial movement of the instrument 41, the leaves 116, 118 are brought into engagement with the septum 90 of the valve 45. In this position, the septum 90 retards axial movement of the adjuster 114 so that further insertion of the instrument 41 tends to cause the leaves 116, 119 to expand outwardly. With the adjuster 114 in contact with the septum 90, its outward expansion together with further axial movement causes the septum 90 to stretch outwardly thereby enlarging the orifice 50. This of course reduces frictional drag on the instrument 41.

In this second position, the adjuster 114 is disposed between the instrument 41 and at least a portion of the septum 90. In a particular embodiment, it may be desirable that the adjuster 114 extend entirely between the instrument 41 and the septum 90 of that the valve 45 is completely removed from contact with the instrument 41. Such an embodiment is illustrated in Figure 3 where contact between the valve 45 and the instrument 41 is inhibited by providing the leaves 116, 118 with an axial extension in the second position which is equal to about the length of the septum 90.

In this particular aspect of the invention, which involves the insertion of an instrument having a diameter greater than that of the orifice 52, the insufflation seal is formed by the valve 47 while the valve 45 is at least partially displaced from contact with the instrument 41.

Once the instrument 41 is operatively disposed, it may be desirable to release the septum 90 so that it can form a second seal with the instrument 41. This can be accomplished by moving the valve adjuster 114 from the second position back to the first position thereby permitting the septum 90 to contact the instrument 41 slightly thereby applying a proximal force to the adjuster 114. This movement from the second position to the first position may also be facilitated by placing a compression spring 130 in the guideway 110 to seat against the shoulder 108 of the separator 105 and the opposing shoulder 123 of the support 121.

A preferred method associated with the invention involves a trocar having at least one valve such as the valve 45 disposed in the housing 25. Such a method might include the step of providing a valve adjuster 114 movable from the first position spaced from the seal 45 to a second position in proximity to the seal 45. In this second position, the valve adjuster 114 would be disposed to at least enlarge the orifice 50 of the valve 45 and perhaps entirely remove the septum 90 from the working channel 30. Then by inserting an instrument 41 into the working channel, it would initially engage the valve adjuster 114 at the first position and then move the adjuster axially from the first position to the second position.

During movement along this path, the method may include the steps of initially contacting the valve adjuster 114 in a relaxed state and then sliding the adjuster from the first position into contact with the septum of the valve 45. After engaging the septum 90, with the adjuster 114 typically in the relaxed state, further axial movement of the instrument 41 would result in expanding the adjuster 114 and the septum 90 radially outwardly to enlarge the orifice 50.

In an embodiment wherein the valve 45 is totally isolated from the instrument 41, the method may include the step of withdrawing the instrument to displace the adjuster 114 from the second position. This step of withdrawing could be facilitated by biasing the adjuster 114, for example with the spring 130, to move the adjuster 114 from the second position back to the first position.

In a further embodiment of the invention illustrated in Figure 4, the trocar 10 includes but a single valve 45. Even in this embodiment, the advantages associated with the valve adjuster 114 are apparent. As previously discussed, an instrument, such as the instrument 41, can be inserted into the trocar 10 to engage the valve adjuster 114. Further movement of the instrument can move the septum 90 associated with the valve 45, enlarging the orifice 50 and inhibiting the frictional engagement of the instrument 41 by the seal 45. In this manner, the valve 45 is protected from any sharp configuration of the instrument 41 until the instrument had been fully inserted. Then the adjustor 114 can be moved back to the first position to create the desired seal between the valve 45 and the instrument 41. In this embodiment, the separator 105 abuts the flange 85 of the cover 82. In addition, the annular support 121 is positioned against the flange 85 when the valve adjuster 114 is in the first position.

The valve adjuster 114 could also be provided with many different configurations, as long as it performs the function of displacing the septum 90 during insertion of the instrument 41 to limit friction or otherwise protect the valve 45.

Given the wide variation within this concept, one is cautioned not to restrict the invention to the embodiments which have been specifically disclosed and illustrated, but rather encouraged to determine the scope of the invention only with reference to the following claims.

## Claims

1. A trocar adapted to provide a working channel across a body wall to accommodate a surgical instrument (41) having a particular diameter, the trocar comprising:
a cannula (16) defining the working channel (30) along an axis extending between a distal end and a proximal end of the cannula (16);
a housing (25) attached to the cannula (16) at the proximal end of the cannula (16);
a valve (45) disposed in the housing (25) so that when engaged by the instrument (41) it forms a fluid tight seal across the working channel;
a valve adjustment means (114) disposed within the housing (25) and having at least a portion disposed in the working channel (30) proximally of the valve (45),
**characterised by**
the valve adjustment means (14) being adapted to move axially within said working channel (30) between a first position spaced from the valve (45) and a second position wherein at least a portion of the valve adjustment means (114) is interposed between the valve (45) and a portion of the working channel (30) which extends through the valve (45), such that the valve (45) is protected from the instrument (41) when the distal tip of the instrument (41) is inserted therethrough; and
the valve adjustment means (114) being responsive to insertion of the instrument (41) into the working channel to move from the first position to the second position.

2. A trocar according to Claim 1, **characterised by** means defining a guideway (110) for guiding the valve adjustment means (114) during movement of the valve adjustment means (114) between the first position and the second position.

3. A trocar according to Claim 2, **characterised in that** the guideway (110) is disposed axially of the trocar so that the first position of the valve adjustment means (114) is spaced axially from the second position of the valve adjustment means (114).

4. A trocar according to Claim 1, **characterised in that** the valve means (114) comprises:
a wall (118) defining a portion of the working channel (30) which extends through the valve adjustment means (114);
a continuous inner surface of the wall (118) having a conical configuration and converging with progressive distal positions along the axis of the cannula (16); whereby
the conical inner surface of the wall (118) of the valve adjustment means (114) is adapted to be contacted by the instrument (41) when the instrument (41) is inserted into the working channel (30).

5. A trocar according to Claim 4, **characterised in that** the wall (118) of the valve adjustment means is radially expandable to enlarge the working channel through the valve adjustment means (114) to a diameter sufficient to permit passage of the instrument (41) through the valve (45).

6. A trocar according to Claim 5, **characterised in that** axial movement of the instrument (41) through the valve adjustment means (114) produces on the valve adjustment means (114) an axial force component tending to move the valve adjustment means (114) axially between the first position and the second position, and a radial force component tending to radially expand the valve adjustment means (114) between the first position and the second position.

7. A trocar according to Claim 1, **characterised in that** the valve adjustment means has a conical inner surface adapted to be engaged by the instrument when the instrument is inserted into the working channel.

8. A trocar according to any preceding claim, wherein the valve comprises a first septum valve having an orifice with a first diameter and further **characterised by** a second septum valve (47) disposed proximally of the first septum valve (45) and within the housing (25) on the opposite side of the valve adjustment means (114) to the first septum valve (45) and having an orifice with a second diameter which is greater than the diameter of orifice in the first septum valve whereby to provide a fluid tight seal across the working channel (30) when the second septum valve (47) is in engagement with the instrument (41) having a particular diameter greater than the second diameter.

9. A trocar recited in any preceding claim, further comprising means for biasing the valve adjustment means to the first position.

10. A trocar according to Claim 8, wherein the valve adjustment means (114) is dimensioned and arranged to remain in the first position in response to insertion of an instrument having a diameter less than that of the orifice of the second septum valve (47).

11. A trocar according to claim 8, **characterised in that** valve adjustment means (114) acts to enlarge the orifice in the first septum valve (45) as the valve adjustment means (114) moves from the first position to the second position.

12. A method for protecting a valve (45) disposed in a trocar having an axis, the trocar having a working channel (30) extending through an orifice in the valve (45) the method comprising the steps of:
providing in the trocar, a valve adjuster (114) movable from a first position spaced from the valve (45) to a second position in contact with the valve (45), the valve adjuster (114) in the second position being disposed to enlarge the orifice of the valve (45);
inserting an instrument (41) into the working channel of the trocar, the instrument having an elongate shaft and a sharp or puncture prone configuration;
during the inserting step engaging the valve adjuster (114) at the first position with the instrument;
moving the valve adjuster (114) axially with the instrument from the first position to the second position; and
radially expanding the valve adjuster (114) and the valve (45) to enlarge the orifice of the valve (45).

13. A method according to Claim 12, **characterised by** the step of biasing the valve adjuster to the first position.

## Patentansprüche

1. Trocar, welcher dazu ausgelegt ist, einen Arbeitskanal durch eine Körperwand hindurch zu schaffen, um ein chirurgisches Instrument (41) mit einem bestimmten Durchmesser aufzunehmen, wobei der Trocar umfaßt:
eine Kanüle (16), welche den Arbeitskanal (30) entlang einer Achse definiert, die sich zwischen einem distalen Ende und einem proximalen Ende der Kanüle (16) erstreckt;
ein Gehäuse (25), welches an der Kanüle (16) am proximalen Ende dieser Kanüle (16) angebracht ist;
ein Ventil (45), welches in dem Gehäuse (25) so angeordnet ist, daß es dann, wenn ein Eingriff des Instrumentes (41) erfolgt, eine fluiddichte Abdichtung über den Arbeitskanal bildet;
Ventileinstellmittel (114), welche innerhalb des Gehäuses (25) angeordnet sind und wenigstens einen Abschnitt aufweisen, welcher in dem Arbeitskanal (30) auf der proximalen Seite von dem Ventil (45) angeordnet ist, **dadurch gekennzeichnet, daß** die Ventileinstellmittel (114) so ausgelegt sind, daß sie sich axial innerhalb des Arbeitskanals (30) zwischen einer ersten Position, die von dem Ventil (45) beabstandet ist, sowie einer zweiten Position bewegen, bei der wenigstens ein Abschnitt der Ventileinstellmittel (114) zwischen dem Ventil (45) und einem Abschnitt des Arbeitskanals (30), der sich durch das Ventil (45) hindurcherstreckt, angeordnet ist derart, daß das Ventil (45) gegenüber dem Instrument (41) geschützt ist, wenn die distale Spitze des Instrumentes (41) durch dieses hindurch eingeführt wird; und
wobei die Ventilelinstellmittel (114) auf ein Einführen des Instrumentes (41) in den Arbeitskanal ansprechen und sich aus der ersten Position in die zweite Position bewegen.

2. Trocar nach Anspruch 1, **gekennzeichnet durch** Mittel, welche eine Führung (110) zum Führen der Ventileinstellmittel (114) während der Bewegung der Ventileinstellmittel (114) zwischen der ersten Position und der zweiten Position definieren.

3. Trocar nach Anspruch 2, **dadurch gekennzeichnet, daß** die Führung (110) so axial zu dem Trocar angeordnet ist, daß die erste Position der Ventileinstellmittel (114) axial von der zweiten Position der Ventileinstellmittel (114) beabstandet sind.

4. Trocar nach Anspruch 1, **dadurch gekennzeichnet, daß** die Ventileinstellmittel (114) umfassen:
eine einen Abschnitt des Arbeitskanals (30) definierende Wand (118), die sich durch die Ventileinstellmittel (114) hindurch erstreckt;
wobei eine kontinuierliche innere Fläche der Wand (118) eine konische Konfiguration hat und mit zunehmenden distalen Positionen entlang der Achse der Kanüle (116) konvergiert; wodurch
die konische innere Fläche der Wand (118) der Ventileinstellmittel (114) dazu ausgelegt sind, durch das Instrument (41) berührt zu werden, wenn das Instrument (41) in den Arbeitskanal (30) eingeführt wird.

5. Trocar nach Anspruch 4, **dadurch gekennzeichnet, daß** die Wand (118) der Ventileinstellmittel radial expandierbar ist, um den Arbeitskanal durch die Ventileinstelimittel (114) auf einen Durchmesser aufzuweiten, welcher ausreicht, um einen Durchtritt des Instrumentes (41) durch das Ventil (45) hindurch zu ermöglichen.

6. Trocar nach Anspruch 5, **dadurch gekennzeichnet, daß** eine axiale Bewegung des Instrumentes (41) durch die Ventileinstellmittel (114) auf die Ventileinstellmittel (114) eine axiale Kraftkomponente aufbringt, welche die Tendenz hat, die Ventileinstellmittel (114) axial zwischen der ersten Position und der zweiten Position zu bewegen, sowie eine radiale Kraftkomponente, welche die Tendenz hat, die Ventileinstellmittel (114) radial zwischen der ersten Position und der zweiten Position zu expandieren.

7. Trocar nach Anspruch 1, **dadurch gekennzeichnet, daß** die Ventileinstellmittel eine konische innere Fläche haben, die dazu ausgelegt ist, daß sich das Instrument an diese anlegt, wenn das Instrument in den Arbeitskanal eingeführt wird.

8. Trocar nach einem der vorangehenden Ansprüche, bei welchem das Ventil ein erstes Septumventil umfaßt, welches eine Mündung mit einem ersten Durchmesser hat, und weiter **gekennzeichnet durch** ein zweites Septumventil (47), welches auf der proximalen Seite von dem ersten Septumventil (45) und innerhalb des Gehäuses (25) an der von dem ersten Septumventil (45) abgewandten Seite der Ventileinstellmittel (114) angeordnet ist und welches eine Mündung mit einem zweiten Durchmesser hat, der größer als der Durchmesser der Mündung in dem ersten Septumventil ist, um **dadurch** eine fluiddichte Abdichtung über den Arbeitskanal (30) zu schaffen, wenn das zweite Septumventil (47) sich im Eingriff mit dem Instrument (41) befindet, welches einen bestimmten Durchmesser größer als der zweite Durchmesser hat.

9. Trocar nach einem der vorangenenden Ansprüche, ferner umfassend Mittel zum Vorspannen der Ventileinstellmittel in die erste Position.

10. Trocar nach Anspruch 8, bei welchem die Ventileinstellmittel (114) so dimensioniert und angeordnet sind, daß sie in Reaktion auf die Einführung eines Instrumentes mit einem Durchmesser, der kleiner als derjenige der Mündung des zweiten Septumventils (47) ist, in der ersten Position verbleibt.

11. Trocar nach Anspruch 8, **dadurch gekennzeichnet, daß** Ventileinstellmittel (114) bewirken, daß die Mündung in dem ersten Septumventil (45) aufgeweitet wird, wenn die Ventileinstellmittel (114) sich aus der ersten Position in die zweite Position bewegen.

12. Verfahren zum Schützen eines in einem Trocar mit einer Achse angeordneten Ventils (45), wobei der Trocar einen Arbeitskanal (30) hat, der sich durch eine Mündung in dem Ventil (45) hindurch erstreckt, wobei das Verfahren die folgenden Schritte umfaßt:
Vorsehen eines Ventileinstellers (114) in dem Trocar, welcher aus einer ersten Position, die von dem Ventil (45) beabstandet ist, in eine zweite Position im Kontakt mit dem Ventil (45) bewegbar ist, wobei der Ventileinsteller (114) in der zweiten Position so angeordnet ist, daß er die Mündung des Ventils (45) aufweitet;
Einführen eines Instrumentes (41) in den Arbeitskanal des Trocar, wobei das Instrument einen langgestreckten Schaft und eine scharf oder punktförmig abgeschrägte Konfiguration hat;
während des Einführschrittes Ineingriffbringen des Instrumentes mit dem Ventileinsteller (114) bei der ersten Position;
Bewegen des Ventileinstellers (114) axial mit dem Instrument aus der ersten Position in die zweite Position; und
Expandieren des radialen Ventileinstellers (114) und des Ventils (45), um die Mündung des Ventils (45) aufzuweiten.

13. Verfahren nach Anspruch 12, **gekennzeichnet durch** den Schritt des Vorspannens des Ventileinstellers in die erste Position.

## Revendications

1. Trocart conçu pour fournir un canal de travail dans la paroi d'un corps pour loger un instrument chirurgical (41) ayant un diamètre particulier, ce trocart comprenant :
une canule (16) définissant le canal de travail (30) le long d'un axe qui s'étend entre une extrémité distale et une extrémité proximale de la canule (16) ;
un boîtier (25) fixé sur la canule (16) au niveau de l'extrémité proximale de la canule (16) ;
une valve (45) disposée dans le boîtier (25) de telle façon que, lorsqu'un instrument (41) est engagé dans celle-ci, elle forme un joint étanche au fluide sur l'intégralité du canal de travail ;
un moyen d'ajustement de la valve (114) disposé à l'intérieur du boîtier (25) et ayant au moins une partie disposée dans le canal de travail (30) à proximité de la valve (45), **caractérisé en ce que** :
le moyen d'ajustement de la valve (114) est conçu pour se déplacer axialement à l'intérieur dudit canal de travail (30) entre une première position espacée de la valve (45) et une seconde position dans laquelle au moins une partie du moyen d'ajustement de la valve (114) est interposée entre la valve (45) et une partie du canal de travail (30) qui s'étend au travers de la valve (45), de telle sorte que la valve (45) est protégée de l'instrument (41) lorsque le bout distal de l'instrument (41) est inséré à travers celle-ci ; et
le moyen d'ajustement de la valve (114) réagit à l'insertion de l'instrument (41) dans le canal de travail en se déplaçant de la première position à la seconde position.

2. Trocart selon la revendication 1, **caractérisé par** un moyen définissant un passage de guidage (110) permettant de guider le moyen d'ajustement de la valve (114) au cours du mouvement du moyen d'ajustement de la valve (114) entre la première position et la seconde position.

3. Trocart selon la revendication 2, **caractérisé en ce que** le passage de guidage (110) est disposé axialement par rapport au trocart, de telle sorte que la première position du moyen d'ajustement de la valve (114) est espacée axialement de la seconde position du moyen d'ajustement de la valve (114).

4. Trocart selon la revendication 1, **caractérisé en ce que** le moyen d'ajustement de la valve (114) comprend :
une paroi (118) définissant une partie du canal de travail (30) qui s'étend au travers du moyen d'ajustement de la valve (114) ;
une surface interne continue de la paroi (118) ayant une configuration conique et convergeant avec des positions distales progressives le long de l'axe de la canule (16) ; grâce à quoi
la surface interne conique de la paroi (118) du moyen d'ajustement de la valve (114) est conçue pour venir en contact avec l'instrument (41) lorsque l'instrument (41) est inséré dans le canal de travail (30).

5. Trocart selon la revendication 4, **caractérisé en ce que** la paroi (118) du moyen d'ajustement de la valve peut s'allonger radialement pour agrandir le canal de travail au travers du moyen d'ajustement de la valve (114) jusqu'à atteindre un diamètre suffisant pour permettre le passage de l'instrument (41) au travers de la valve (45).

6. Trocart selon la revendication 5, **caractérisé en ce que** le mouvement axial de l'instrument (41) au travers du moyen d'ajustement de la valve (114) produit, sur le moyen d'ajustement de la valve (114), une composante de force axiale qui tend à déplacer le moyen d'ajustement de la valve (114) axialement entre la première position et la seconde position, ainsi qu'une composante de force radiale qui tend à expandre radialement le moyen d'ajustement de la valve (114) entre la première position et la seconde position.

7. Trocart selon la revendication 1, **caractérisé en ce que** le moyen d'ajustement de la valve (114) a une surface interne conique conçue pour qu'un instrument s'y engage lorsque l'instrument est inséré dans le canal de travail.

8. Trocart selon l'une quelconque des revendications précédentes, dans lequel la valve comprend une première valve de cloison (45) ayant un orifice ayant un premier diamètre, et **caractérisé en outre par** une seconde valve de cloison (47) disposée à proximité de la première valve de cloison (45) et à l'intérieur du boîtier (25) sur le côté opposé du moyen d'ajustement de la valve (114) par rapport à la première valve de cloison (45), et ayant un orifice ayant un second diamètre qui est supérieur au diamètre de l'orifice pratiqué dans la première valve de cloison (45), ce qui permet de fournir un joint étanche au fluide sur l'intégralité du canal de travail (30) lorsque la seconde valve de cloison (47) se trouve en engagement avec l'instrument (41), et ayant un diamètre particulier supérieur au second diamètre.

9. Trocart selon l'une quelconque des revendications précédentes, comprenant en outre un moyen de sollicitation du moyen d'ajustement de la valve (114) vers la première position.

10. Trocart selon la revendication 8, dans lequel le moyen d'ajustement de la valve (114) présente des dimensions et est agencé pour rester dans la première position en réponse à l'insertion d'un instrument ayant un diamètre inférieur à celui de l'orifice de la seconde valve de cloison (47).

11. Trocart selon la revendication 8, **caractérisé en ce que** le moyen d'ajustement de la valve (114) agit pour élargir l'orifice de la première valve de cloison (45) au moment où le moyen d'ajustement de la valve (114) se déplace de la première position vers la seconde position.

12. Procédé de protection d'une valve (45) disposée dans un trocart ayant un axe, ce trocart ayant un canal de travail (30) qui s'étend au travers d'un orifice pratiqué dans la valve (45), ledit procédé comprenant les étapes consistant à :
fournir dans le trocart un appareil d'ajustement de la valve (114) qui peut se déplacer d'une première position espacée de la valve (45) vers une seconde position en contact avec la valve (45), l'appareil d'ajustement de la valve (114), dans la seconde position, étant disposé pour élargir l'orifice de la valve (45) ;
insérer un instrument (41) dans le canal de travail du trocart, l'instrument ayant un arbre allongé et une configuration de préférence de perforation ou pointue ;
au cours de l'étape d'insertion, engager l'appareil d'ajustement de la valve (114) dans la première position, avec l'instrument ;
déplacer l'appareil d'ajustement de la valve (114) axialement avec l'instrument, de la première position vers la seconde position ; et
expandre radialement l'appareil d'ajustement de la valve (114) pour élargir l'orifice de la valve (45).

13. Procédé selon la revendication 12, **caractérisé par** l'étape de sollicitation de l'appareil d'ajustement de la valve (114) vers la première position.
